# EUROPÄISCHE PATENTSCHRIFT

(11) **EP 4 472 706 B1**
(45) Veröffentlichungstag und Bekanntmachung des Hinweises auf die Patenterteilung: **08.04.2026**
(21) Anmeldenummer: 23703042.4
(22) Anmeldetag: 01.02.2023
(51) Int. Cl.: A61M 16/04

(54) **SCHILD FÜR TRACHEOSTOMIEKANÜLE**
SHIELD FOR A TRACHEOSTOMY CANNULA
PROTECTION POUR CANULE DE TRACHÉOTOMIE

(30) Priorität: 04.02.2022 DE 102022102715
(43) Veröffentlichungstag der Anmeldung: 11.12.2024
(73) Patentinhaber: Coloplast A/S, 3050 Humlebaek (DK)
(72) Erfinder: SCHNELL, Ralf, 63500 Seligenstadt (DE)
(86) Internationale Anmeldenummer: PCT/EP2023/052449
(87) Internationale Veröffentlichungsnummer: WO 2023/148224

(56) Entgegenhaltungen:
- DE-U1- 202010 004 297
- US-A- 5 398 679
- US-A- 5 975 080

## Beschreibung

Die vorliegende Offenbarung betrifft einen Schild zur Anbringung an einem Kanülenrohr einer Tracheostomiekanüle optional über einen an dem Kanülenrohr angeordneten ringförmigen Konnektor, wobei der Schild einen zentralen Bereich und zwei Seitenteile aufweist, die sich an zwei gegenüberliegenden Seiten des zentralen Bereichs anschließen und sich von dort in radialer Richtung bis zum jeweiligen Ende des Seitenteils erstrecken, wobei die beiden Seitenteile jeweils eine Öse zum Hindurchführen eines Haltebandes aufweisen. Darüber hinaus betrifft die vorliegende Offenbarung eine Tracheostomiekanüle zur Einbringung in ein Tracheostoma mit einem Kanülenrohr und einem an der Kanüle angeordneten Schild der offenbarungsgemäßen Art.

Tracheotomietuben, die häufig auch als Tracheostomiekanülen bezeichnet werden, sind üblicherweise mit einer Halsplatte, die auch als Kanülenschild bezeichnet wird, ausgestattet, damit der Tubus, bzw. die Kanüle nicht tiefer in die Trachea eindringt, als gewünscht. Handelsübliche Kanülenschilder sind typischerweise plattenförmig und weisen an zwei Seiten jeweils eine Öse auf, an der das Kanülenband eingefädelt werden kann. Die Ösen sind dabei typischerweise einfach in Form von durchgehenden Ausnehmungen in dem plattenförmigen Schild vorgesehen.

Das Kanülenband ist erforderlich, um die Kanüle am Patientenhals zu fixieren, damit die Kanüle nicht versehentlich aus dem Tracheostoma herausrutscht. Das Kanülenband wird für diese Zwecke um den Patientenhals geführt und jeweils an den am Ende des linken und rechten Seitenteils angeordneten Ösen des Kanülenschilds befestigt.

Die Befestigung des Kanülenbandes an der Öse kann dadurch erfolgen, dass mit dem Band eine Schlaufe gebildet wird, wobei das Kanülenband über eine Klettverbindung an sich selbst fixiert wird. Alternativ können an den Enden der Kanülenbänder auch Haken zum Einhängen in die Öse vorgesehen sein. Manche Kanülenbänder werden auch einfach an der Öse des Kanülenschildes festgeknotet. Von einigen Anwendern werden die Ösen auch dazu verwendet, um Kanülenschilder am Patientenhals festzunähen.

Häufig führen Kanülenbänder, die durch Ösen am Ende der Seitenteile eines Kanülenschilds eingefädelt werden, zu Hautirritationen. Dies liegt daran, dass die Öse bei vielen Kanülenschilden so angeordnet ist, dass ein Teil des Kanülenbands zwischen der zum Hals des Patienten weisenden Schildfläche und dem Hals des Patienten verläuft und so bei Bewegung des Patienten ständig an der Patientenhaut reibt.

Aus US 8,522,788 B2 ist ein Kanülenschild bekannt, bei dem an den Enden des Schildes eine stufenweise Verdickung vorgesehen ist, in welcher dann eine Öse zum Befestigen eines Haltebandes ausgebildet ist. Bei manchen Ausführungsformen ist die Öse so ausgebildet, dass das Halteband so geführt werden kann, dass es nicht zwischen Schild und Patientenhals verläuft.

In DE 10 2011 006 064 wird ein Kanülenschild beschrieben, bei dem das Kanülenschild an seinen äußersten Enden vom Patientenhals weg aufgewölbt ist, und in diesem aufgewölbten Endbereich sind jeweils Halteösen in das Schildmaterial eingelassen. Durch die Aufwölbung an den Enden der Seitenteile liegt die Öse nicht auf der Höhe des Patientenhalses, sodass das Halteband so geführt werden kann, dass es nicht zwischen Schild und Patientenhals verläuft.

Die beiden vorgenannten Druckschriften aus dem Stand der Technik lösen das Problem der Hautirritationen nur teilweise, indem das Kanülenband etwas oberhalb des Hautniveaus durch die Öse geführt wird. Insbesondere bei adipösen Patienten drückt das Kanülenband das Kanülenschild allerdings oft so tief in das weiche Gewebe ein, dass das Kanülenband doch noch Kontakt zur Haut hat, und die Kanülenöse liegt dann auch nicht immer so frei, dass man ein neues Kanülenband einfädeln kann, ohne dass Schild leicht anzuheben. Letzteres ist für den Patienten meist sehr unangenehm und führt zu Hustenreiz oder auch zu Würgereiz. Darüber hinaus hat sich gezeigt, dass bei der Befestigung des Kanülenbandes an seitlichen Ösen oberhalb des Hautniveaus bei einseitigen seitlichen Belastungen und bei bestimmten Patientenanatomien gewisse Hebel- und Kippeffekte feststellbar sind, die zu einem Verkippen des Schildes führen, was für den Patienten recht unangenehm sein kann. Dies ist insbesondere bei besonders schlanken Patienten zu beobachten, wenn das Kanülenband einseitig belastet wird.

Die folgenden Dokumente offenbaren auch Schilder für Tracheostomiekanülen, nämlich US 5 975 080 A und US 5 398 679 A.

Aufgabe der vorliegenden Erfindung war es vor diesem Hintergrund nach einer Lösung zu suchen, mit der sichergestellt werden kann, dass das Kanülenband dort, wo es in die Öse eingefädelt wird, keinen Hautkontakt hat, ohne das es zu unerwünschten Hebel- und Kippeffekten beim Anlegen und Befestigen des Schildes kommen kann.

Diese Aufgabe wird erfindungsgemäß durch die Merkmale des Anspruchs 1 gelöst.

Die Begriffe "distal" und "proximal" werden im Sinne der vorliegenden Erfindung aus der Sicht eines die Beatmungsvorrichtung anwendenden Arztes verwendet, d. h. das proximale Ende ist das Ende der Beatmungsvorrichtung, das nach dem Einführen in die Trachea außerhalb des Patientenkörpers verbleibt, während das distale Ende in die Trachea des Patienten eingeführt wird.

Dadurch, dass bei der vorliegenden Offenbarung die jeweils eine Öse an den Seitenteilen des Schildes von einem brückenartigen Befestigungselement gebildet wird, dessen Pfeilerabschnitte auf der proximalen Fläche des Schildes in einem Abstand vom Ende des jeweiligen Seitenteils angeordnet sind, wird verhindert, dass ein Teil des an der Öse befestigten Kanülenbandes zwischen Schild und Haut eingeklemmt werden kann und so Hautirritationen auslösen kann.

Darüber hinaus können beim Anlegen und beim Einführen und Befestigen des Kanülenbandes auf diese Art und Weise deutlich seltener ungünstige Hebel- und Kippeffekte auftreten, die zu einem Verkippen des Schildes auf dem Hals des Patienten führen können. Jedenfalls ist die

Kippneigung durch die offenbarungsgemäß vorgeschlagene Anordnung eines brückenartigen Befestigungselements in einem Abstand zum Ende der Seitenteile deutlich geringer im Vergleich zu Schilden, bei denen die Ösen für das Halteband am äußersten Ende der Seitenteile des Schildes angeordnet sind.

Bei bestimmten Ausführungsformen stehen die Pfeilerabschnitte des brückenartigen Befestigungselementes in demselben Abstand vom äußersten Ende des jeweiligen Seitenteiles auf der proximalen Fläche des Schildes auf. Bei alternativen Ausführungsformen stehen die Pfeilerabschnitte des brückenartigen Befestigungselementes in unterschiedlichen Abständen vom äußersten Ende des jeweiligen Seitenteiles auf. Der Abstand zum Ende des jeweiligen Seitenteiles hängt unter anderem davon ab, wie der Schild geometrisch zugeschnitten ist. Bezogen auf eine Projektion der zentralen Längsachse des Seitenteiles auf dessen proximale Fläche können die Pfeilerabschnitte eines Befestigungselementes auf einer Linie aufstehen, die auf der Längsachse oder parallel dazu verläuft. Unter der zentralen Längsachse ist hier die zentrale Achse des Schildes in Längsrichtung vom äußeren Ende des einen Seitenteils zum äußeren Ende des anderen Seitenteils zu verstehen. Bei alternativen Ausführungsformen stehen die Pfeilerabschnitte eines Befestigungselementes auf der proximalen Fläche des Seitenteiles auf einer Linie, welche die Projektion der zentralen Längsachse des Seitenteiles auf dessen proximale Fläche in einem Winkel von 45 bis 90° schneidet.

Bei manchen Ausführungsformen können die beiden Pfeilerabschnitte des auf einem Seitenteil angeordneten brückenartigen Befestigungselements bezogen auf die Projektion der zentralen Längsachse des Seitenteiles auf dessen proximale Fläche spiegelsymmetrisch angeordnet sein. Je nach Ausführungsform, kann von dieser Form der Symmetrie auch abgewichen werden. Die beiden auf den zwei Seitenteilen des Schildes angeordneten Befestigungselemente sind bei bestimmten Ausführungsformen bezogen auf eine Projektion der zentralen Querachse des Schildes spiegelsymmetrisch angeordnet.

Die beiden Pfeilerabschnitte des brückenartigen Befestigungselementes und der sich dazwischen erstreckende Überbrückungsabschnitt bilden zusammen mit der proximalen Fläche des Seitenteiles eine Öse, durch die ein Halteband geführt und/oder an der das Halsband befestigt werden kann. Bei bestimmten Ausführungsformen beträgt der Abstand zwischen den Innenseiten der Pfeilerabschnitte eines brückenartigen Befestigungselements 3 bis 12 mm. Die lichte Höhe zwischen der proximalen Fläche des Seitenteiles und der Innenseite des Überbrückungsabschnitts liegt vorzugsweise im Bereich von 2 bis 5 mm. Mit diesen Maßen wird eine Öse gebildet, durch welche die meisten handelsüblichen Haltebänder gut hindurchführbar sind.

Um die Höhe, mit der das brückenartige Befestigungselement über die proximale Fläche des Schildes erhaben ist, möglichst gering zu halten, ist bei manchen Ausführungsformen unter dem brückenartigen Befestigungselement eine Ausnehmung in der proximalen Fläche vorgesehen. Bei einigen Ausführungsformen ist die Ausnehmung unter dem brückenartigen Befestigungselement muldenförmig ausgeführt. Bei anderen Ausführungsformen ist diese Ausnehmung nutartig bis zum Ende des Seitenteils durchgehend ausgeführt. Bei den Ausführungsformen mit solchen Ausnehmungen wird die lichte Höhe zwischen der proximalen Fläche des Seitenteiles und der Innenseite des Überbrückungsabschnitts auf die tiefste Stelle der nutartigen oder muldenartigen Ausnehmung bezogen.

An dem in Richtung des zentralen Bereichs weisenden Ende der nutartigen Ausnehmung, steigt der Nutboden bei bestimmten Ausführungsformen bis auf das Niveau der proximalen Fläche an. Ein in diese Richtung eingeführtes Halteband wird somit in proximale Richtung geführt und kann damit vom Anwender leichter gegriffen werden.

Um das Risiko einer Kippbelastung bei einseitigem Zug auf das Kanülenband besonders effektiv zu reduzieren, beträgt der minimale Abstand der Pfeilerabschnitte vom Ende des jeweiligen Seitenteiles in einer bevorzugten Ausführungsform wenigstens 2 mm. Noch bessere Ergebnisse können mit bestimmten Ausführungsformen erzielt werden, bei denen der minimale Abstand wenigstens 4 mm beträgt. Bei besonderen Ausführungsformen beträgt der minimale Abstand der Pfeilerabschnitte vom Ende des jeweiligen Seitenteiles wenigstens 6 mm.

Die Geometrie des brückenartigen Befestigungselements ist relativ variabel. Insbesondere umfasst sind von der vorliegenden Erfindung brückenartige Befestigungselemente, bei denen die beiden Pfeilerabschnitte des jeweiligen Befestigungselements mit dem sich zwischen den beiden Pfeilerabschnitten erstreckenden Überbrückungsabschnitt jeweils einen Winkel im Bereich von 90° und 135° bilden oder die beiden Pfeilerabschnitte des jeweiligen Befestigungselements mit dem sich zwischen den beiden Pfeilerabschnitten erstreckenden Überbrückungsabschnitt einen Rundbogen beschreiben. Umfasst sind auch solche Ausführungsformen, bei denen die Oberseite des brückenartigen Befestigungselements eckig ausgeführt ist und die in Richtung der Ösenöffnung weisende Innenseite als Rundbogen ausgeführt ist und andersherum. Der Begriff "eckig" soll ist hier so zuverstehen, dass hiervon auch Ausführungsformen mit Ecken umfasst sind, die abgerundet oder gefast sind, um scharfe Kanten oder Ecken zu vermeiden.

Bei bestimmten Ausführungsformen haben die beiden Pfeilerabschnitte des jeweiligen Befestigungselementes einen rechteckigen oder einen abgerundet rechteckigen Querschnitt. Bei alternativen Ausführungsformen haben die Pfeilerabschnitte einen ovalen oder kreisrunden Querschnitt.

Bei bestimmten Ausführungsformen hat der sich zwischen den beiden Pfeilerabschnitten erstreckende Überbrückungsabschnitt einen rechteckigen oder einen abgerundet rechteckigen Querschnitt. Bei alternativen Ausführungsformen hat der Überbrückungsabschnitt einen ovalen oder kreisrunden Querschnitt.

Insgesamt gilt, dass abgerundete Kanten und Ecken unter hygienischen Gesichtspunkten Vorteile bieten. Eher eckige Ausführungsvarianten können unter Umständen Vorteile im Hinblick auf Befestigung und Haltefunktion bieten, je nachdem welche Befestigungstechnik für das Halteband zum Einsatz kommt.

Damit man das Ende des Haltebands nach dem Einfädeln durch die Öse gut greifen kann ist bei bestimmten Ausführungsformen auf der proximalen Fläche zwischen der Öse und dem zentralen Bereich ein Anhebeelement vorgesehen, das eine Anhebefläche bereitstellt, die auf der Seite zur Öse hin mit der proximalen Fläche im Wesentlichen bündig abschließt und in Richtung zentraler Bereich um 1 bis 5 mm oder 1 bis 3 mm ansteigt, wodurch das Ende eines aus der Richtung des Seitenteilendes in Richtung des zentralen Bereichs durch die Öse geführten Haltebandes von der proximalen Fläche des Seitenteils leicht abgehoben wird, so dass man dieses Ende leichter greifen kann.

Bei bestimmten Ausführungsformen stehen die beiden Pfeilerabschnitte des jeweiligen Befestigungselements in einem rechten Winkel auf der proximalen Fläche des Schildes auf. Bei alternativen Ausführungsformen stehen die Pfeilerabschnitte in einem Winkel, der um +/-45° vom rechten Winkel abweicht auf. Die Abweichung vom rechten Winkel ist bei den beiden Pfeilerabschnitten eines Befestigungselementes bei bestimmten Ausführungsformen entweder absolut oder zumindest betragsmäßig gleich. Darüber hinaus können die beiden Pfeilerabschnitte eines Befestigungselementes auch bezogen auf die zentrale Längsachse des zentralen Bereiches entweder in radialer Richtung oder in tangentialer Richtung abweichen.

Das auf den Seitenteilen offenbarungsgemäß vorgesehene brückenartige Befestigungselement stellt eine Öse bereit, durch die das Halteband geführt und an der das Halteband befestigt werden kann. Da es jedoch auch Anwender gibt, die die Möglichkeit haben möchten, das Kanülenband je nach den gegebenen Umständen alternativ durch eine herkömmliche Öse einfädeln zu können, ist bei bestimmten Ausführungsformen der vorliegenden Offenbarung zusätzlich zu der durch das brückenartige Befestigungselement gebildeten Öse eine herkömmliche Öse vorgesehen, die in der Schildfläche in dem Bereich vorgesehen ist, der von dem brückenartigen Befestigungselement überspannt wird. Die Seitenteile weisen an dieser Stelle also von der proximalen Fläche bis zur distalen Fläche durchgängige Ausnehmungen auf, die beispielsweise rechteckig, kreisrund, oval oder als Langloch ausgebildet sein können. Bei den Ausführungsformen mit solchen Ausnehmungen wird die lichte Höhe zwischen der proximalen Fläche des Seitenteiles und der Innenseite des Überbrückungsabschnitts auf die Ebene der proximalen Fläche im Randbereich der Ausnehmung bezogen.

Die durchgängigen Ausnehmungen können, wenn sie in einer ersten Dimension auf der Ebene der proximalen Fläche des Seitenteiles eine größere Ausdehnung haben als in einer zweiten Dimension, ähnlich ausgerichtet sein, wie dies bereits für die brückenartigen Befestigungselemente beschrieben wurde. In Längsrichtung der Ausnehmung können sich diese beispielsweise auf einer Linie erstrecken, welche entweder zu einer Projektion der zentralen Längsachse des Seitenteiles auf dessen proximale Fläche parallel verläuft. Alternativ kann die Längsrichtung der Ausnehmung auch auf einer Linie liegen, die die Projektion der zentralen Längsachse des Seitenteiles auf dessen proximale Fläche in einem Winkel von 45 bis 90° schneidet.

Die durchgängigen Ausnehmungen durch das Kanülenschild gehen in einer bevorzugten Ausführungsform in Richtung des Endes des Seitenteils um circa 1 bis 3 mm über den Bereich hinaus, der von dem brückenartigen Befestigungselement überspannt wird. Dies erleichtert die Einfädelung des Kanülenbandes bzw. auch gegebenenfalls das Annähen an den Patientenhals.

Die Linie, auf der sich eine längliche Ausnehmung in deren Längsrichtung auf der proximalen Fläche erstreckt, liegt entweder auf der Linie, auf der die Pfeilerabschnitte des jeweiligen Befestigungselementes auf der proximalen Fläche aufstehen, oder sie verläuft parallel hierzu oder sie schneidet diese auf der in radialer Richtung äußeren Seite des Befestigungselements in einem Winkel von 5 bis 60°, vorzugsweise in einem Winkel von 15 bis 45°. Bezogen auf die zentrale Querachse des Schildes können die beiden durchgängigen Ausnehmungen des Schildes spiegelsymmetrisch angeordnet sein oder nicht spiegelsymmetrisch.

Bei bestimmten Ausführungsformen verläuft die Seitenfläche, die sich zwischen der distalen Fläche und der proximalen Fläche eines Seitenteiles erstreckt, am Ende des Seitenteiles über die Breite des Schildes auf einer bogenförmigen Bahn, sodass die Enden der Seitenteile abgerundet sind. Bei manchen Ausführungsformen sind die Enden des Seitenteiles auch in proximaler Richtung bogenförmig aufgewölbt, sodass am Ende des Seitenteiles keine Kante vorliegt, die am Hals des Patienten zum Aufliegen kommen kann.

Um insgesamt Kanten, die am Hals des Patienten zum Aufliegen kommen können, zu vermeiden, gehen bei bestimmten Ausführungsformen der Offenbarung die Seitenflächen, die sich zwischen der distalen Fläche und der proximalen Fläche eines Seitenteiles erstrecken, ohne Ausbildung einer Kante fließend in die distale Fläche und wahlweise auch in die proximale Fläche des jeweiligen Seitenteiles über.

Das offenbarungsgemäße Schild besteht typischerweise aus einem Kunststoffmaterial. Bei bestimmten Ausführungsformen besteht der Schild aus einem weichen, elastisch verformbaren Material, wie z.B. aus Weich-PV, Polyurethan, SEBS oder einem anderen thermoplastischen Elastomer oder aus Silikon. Bei alternativen Ausführungsformen besteht der Schild aus einem harten, formstabilen Material, wie z.B. Polystyrol, Polycarbonat, ABS, Polyamid oder Polyester. Bei besonderen Ausführungsformen besteht der Schild zum einen Teil aus einem harten, formstabilen Material und zum anderen Teil aus einem weichen, elastisch verformbaren Material.

Bei den Ausführungsformen, bei denen der Schild teilweise aus einem formstabilen und teilweise aus einem weichen, elastisch verformbaren Material besteht, ist die Verteilung der verschiedenen Materialien vorzugsweise wie folgt. Das brückenartige Befestigungselement und der Bereich des Schildes, auf dem das brückenartige Befestigungselement aufsteht, und optional wenigstens abschnittweise auch der Bereich, der um eine gegebenenfalls vorgesehene durchgängige Ausnehmung im Seitenteil des Schildes vorgesehen ist, ist vorzugsweise aus einem harten, formstabilen Material, während die übrigen Bereiche des Schildes aus einem weichen, elastisch verformbaren Material bestehen. Auf diese Weise wird in diesem Bereich die für die Befestigung des Haltebandes erforderliche Stabilität gewährleistet, während gleichzeitig der überwiegende Teil der am Patientenhals anliegenden Flächen aus einem für den Patienten angenehmeren weichen, elastisch verformbaren Material besteht.

Um dem Schild besonders im zentralen Bereich eine höhere Steifigkeit und Stabilität zu verleihen, sind bei bestimmten Ausführungsformen die Bereiche der beiden Schildteile, die im Bereich der Befestigungselemente aus hartem Material bestehen, über eine "Stabilisierungsbrücke" aus demselben harten Material verbunden, wobei sich diese Stabilisierungsbrücke über den zentralen Bereich des Schildes erstreckt.

Unter harten Materialien werden hier Kunststoffe mit einer Shore A Härte ≥90 verstanden, und unter weichen Kunststoffen werden hier solche mit einer Shore A Härte von 30 bis 85 verstanden. Bei den Ausführungsformen, bei denen der Schild aus einem einheitlichen Kunststoffmaterial besteht, ist die Shore A Härte dieses Materials vorzugsweise ≥40, um eine gewisse Mindeststabilität des gesamten Schildes zu gewährleisten.

Die Seitenteile des Schildes sind typischerweise flächig ausgebildet und weisen vorzugsweise eine Materialstärke im Bereich von 1,5 bis 4 mm auf. Die Seitenteile können ausgehend vom zentralen Bereich bis zum Ende der Seitenteile entweder eine durchgängig gleiche oder eine im Verlauf gleichmäßig oder stufenweise abnehmende Materialstärke aufweisen. Die Dicke der Seitenteile des Schildes kann bei bestimmten Ausführungsformen der Offenbarung ausgehend von dem zentralen Bereich bis zum Ende des jeweiligen Seitenteiles aber auch in anderer Weise variieren. Bei den Ausführungsformen mit durchgängig gleich dicken Seitenteilen verläuft die distale Fläche der Seitenteile im Wesentlichen parallel zu der proximalen Fläche des jeweiligen Seitenteiles.

Bei bestimmten Ausführungsformen weist die distale Fläche des offenbarungsgemäßen Schildes im zentralen Bereich einen in Längsrichtung des Schildes in Richtung proximal konkav gekrümmten Abschnitt auf. Bei manchen Ausführungsformen weist die distale Fläche des offenbarungsgemäßen Schildes im zentralen Bereich einen senkrecht zur Längsrichtung des Schildes in Richtung proximal konkav gekrümmten Abschnitt auf. Bei besonderen Ausführungsformen des offenbarungsgemäßen Schildes ist die distale Fläche im zentralen Bereich sowohl in Längsrichtung des Schildes als auch in einer Richtung senkrecht zur Längsrichtung des Schildes in Richtung proximal konkav gekrümmt.

Der durch die konkave Krümmung eines Abschnitts des zentralen Bereichs bedingte größte Abstand der distalen Fläche von einer durch die Scheitelpunkte verlaufenden Tangentialfläche beträgt zwischen 4 mm und 20 mm. Vorzugsweise beträgt der größte Abstand zwischen 6 mm und 12 mm. Auf diese Weise wird besonders gut erreicht, dass am Hals des Patienten keine Druckstellen durch darauf aufliegende Schildabschnitte entstehen.

Um den Schild bei der Anbringung am Hals eines Patienten an die besondere Anatomie des jeweiligen Patienten optimal anpassen zu können, weist der Schild bei bestimmten Ausführungsformen im zentralen Bereich eine Aufhängeinrichtung zur kardanischen Lagerung am Kanülenrohr oder an einem Konnektor einer Tracheostomiekanüle auf.

Ergänzend oder alternativ ist für Zwecke der Anpassung an die besondere Anatomie des jeweiligen Patienten bei bestimmten Ausführungsformen an der distalen Fläche des Schildes anliegend ein separates flächiges Polsterelement aus einem weichen, elastisch verformbaren Material vorgesehen, wobei der Außenumfang des flächigen Polsterelements über den Außenumfang der distalen Fläche des Schildes allseits um wenigstens 1 mm übersteht.

Der erfindungsgemäße Schild wird mit der Kanüle bzw. dem Konnektor verbunden und das Polsterelement lässt sich bei Bedarf vorher aufstecken oder auch nachträglich anbringen. Im Idealfall erfüllt der Schild also seine Funktion mit den weiter oben beschriebenen Eigenschaften auch ohne die hier beschriebene Variante mit einem Polsterelement. Das Polsterelement dient entweder der weiteren Verbesserung der Eigenschaften des erfindungsgemäßen Schildes oder der Individualisierung an die besonderen Anforderungen eines bestimmten Patienten.

Das Polsterelement kann zu einem erfindungsgemäßen Standardschild entweder einzeln oder in Form einer Art Adapter-Satz in verschiedenen Formen und Größen bereitgestellt werden, um dann den Schild über das gewählte Polsterelement *in situ* an die besondere Anatomie und die individuellen Bedürfnisse des jeweiligen Patienten anpassen zu können.

Bei manchen Ausführungsform befindet sich auf der distalen Fläche des Polsterelementes in dem Bereich, der am zentralen Bereich des Schildes zum anliegen kommt, ein konisches Anschlussstück, das in das Stoma eines Patienten eingeführt werden kann um eine bessere Abdichtung zu bewirken. Je nachdem wie groß das Stoma des jeweiligen Patienten ist, können Außendurchmesser, Höhe und Steigung variieren.

Das Material des Polsterelements hat vorzugsweise eine Dicke von wenigstens 0,2 mm, um eine möglichst gute Polsterwirkung zu erzielen. Mit Hilfe des Polsterelementes kann aber auch dafür gesorgt werden, dass eine Kanüle, die für einen bestimmten Patienten etwas zu lang ist, weniger tief in die Trachea eingeführt wird. Das Material des Polsterelements hat daher bei manchen Ausführungsformen eine Dicke von bis zu 12 mm. Bei bestimmten Ausführungsformen liegt die Dicke im Bereich von 0,2 mm bis 8 mm oder im Bereich von 0,2 mm bis 4 mm.

Beispiele für weiche elastomere Materialien, die für das Polsterelement verwendet werden können, wurden oben im Zusammenhang mit weichen Schildbestandteilen bereits genannt.

Bei besonderen Ausführungsformen bildet das Material des Polsterelements einen elastischen Hohlkörper, wodurch die Dicke des Polsterelements durch Befüllung des Hohlkörpers mit unterschiedlichen Volumina eines Füllfluids variiert werden kann. Bei Ausführungsformen mit konischem Anschlussstück kann auch dieses mit einem Hohlkörper versehen sein, wodurch Ausdehnung des Anschlussstücks durch Befüllung des Hohlkörpers mit unterschiedlichen Volumina eines Füllfluids optimal an die Größe des Stomas angepasst werden. Je mehr Fluid man zugibt, desto größer wird der Durchmesser der konischen Abdichtung.

Bei manchen Ausführungsformen sind Schild und Polsterelement miteinander verklebt. Bei alternativen Ausführungsformen wird das Polsterelement am Schild über eine formschlüssige oder kraftschlüssige Verbindung gehalten, wie z.B. einen Schnappverschluss. Bei bestimmten Ausführungsformen sind Schild und Polsterelement durch einen 2K-Spritzguss-Prozess miteinander verbunden.

Eine besondere Ausführungsform dieser Variante ist dadurch gekennzeichnet, dass der Schild aus einem harten Material besteht und in den Seitenteilen des Schildes von der proximalen Fläche bis zur distalen Fläche durchgängige Ausnehmungen vorgesehen sind, die sich wenigstens über den Bereich erstrecken, der sich neben dem von den brückenartigen Befestigungselementen überspannten Bereich erstreckt. Das an der distalen Fläche des Schildes anliegende separate flächige Polsterelement besteht aus einem weichen, elastisch verformbaren Material, wobei der Außenumfang des flächigen Polsterelements über den Außenumfang der proximalen Fläche des Schildes allseits um wenigstens 1 mm übersteht.

Bei der hier beschriebenen Ausführungsform weist das separate Polsterelement zwei Ösen zum Befestigen eines Haltebandes auf, wobei die Ösen jeweils von einem brückenartigen Befestigungselement gebildet werden, das zwei auf der proximalen Fläche des Polsterelements angeordnete Pfeilerabschnitte und einen sich zwischen den beiden Pfeilerabschnitten erstreckenden Überbrückungsabschnitt aufweist. Die beiden brückenartigen Befestigungselemente des Polsterelements sind dabei so vorgesehen, dass sie durch die in den Seitenteilen des Schildes angeordnete durchgängige Ausnehmungen ragen und dadurch so neben den brückenartigen Befestigungselementen des Schildes angeordnet sind, dass die Ösen des Polsterelements mit den Ösen des Schildes im Wesentlichen zur Deckung kommen. Vorzugsweise befinden sich im zusammengesetzten Zustand auch die Pfeiler der Befestigungselemente des Polsterelements in einem Abstand vom Ende des jeweiligen Seitenteiles des Schildes.

Dadurch, dass bei dieser Ausführungsform die Befestigungselemente von Schild und Polsterelement nebeneinander angeordnet sind, kann das Halteband durch jeweils beide Befestigungselemente geführt werden. Auf diese Weise können sich die Komponenten nicht mehr voneinander lösen. Somit kann das Polsterelement nicht versehentlich in die Luftröhre rutschen.

Die vorliegende Erfindung betrifft einen Schild zur Anbringung an einem Kanülenrohr einer Tracheostomiekanüle oder an einem an dem Kanülenrohr proximal angeordneten ringförmigen Konnektor. Von der vorliegenden Erfindung umfasst sind aber auch Tracheostomiekanülen zur Einbringung in ein Tracheostoma mit einem Kanülenrohr und einem Schild der oben beschriebenen erfindungsgemäßen Art.

Für Zwecke der ursprünglichen Offenbarung wird darauf hingewiesen, dass sämtliche Merkmale, wie sie sich aus der vorliegenden Beschreibung, den Zeichnungen und den Ansprüchen für einen Fachmann erschließen, auch wenn sie konkret nur im Zusammenhang mit bestimmten weiteren Merkmale beschrieben wurden, sowohl einzeln als auch in beliebigen Zusammenstellungen mit anderen der hier offenbarten Merkmalen oder Merkmalsgruppen kombinierbar sind, soweit dies nicht ausdrücklich ausgeschlossen wurde oder technische Gegebenheiten derartige Kombinationen unmöglich oder sinnlos machen. Auf die umfassende, explizite Darstellung sämtlicher denkbarer Merkmalskombinationen wird hier nur der Kürze und der Lesbarkeit der Beschreibung wegen verzichtet.

Des Weiteren wird darauf hingewiesen, dass es für den Fachmann selbstverständlich ist, dass die nachfolgenden Ausführungsbeispiele lediglich dazu dienen, die als Ausführungsbeispiele wiedergegebenen möglichen Ausführungsformen der vorliegenden Erfindung beispielhaft anzugeben. Der Fachmann wird daher ohne Weiteres verstehen, dass darüber hinaus auch alle anderen Ausführungsformen, die die in den Ansprüchen genannten erfindungsgemäßen Merkmale oder Merkmalskombinationen aufweisen, innerhalb des Schutzumfangs der Erfindung liegen. Auf die umfassende, explizite Darstellung sämtlicher denkbarer Ausführungsformen wird hier nur der Kürze und der Lesbarkeit der Beschreibung wegen verzichtet.

Einige der oben genannten Merkmale bzw. Merkmalskombinationen sind in den anhängenden Figuren in Form von beispielhaften Ausführungsformen klargestellt, ohne dass die vorliegende Erfindung in irgendeiner Form auf einzelne der hier dargestellten Ausführungsbeispiele beschränkt sein soll.
- Figur 1: zeigt eine Ausführungsform eines an einem Kanülenrohr einer Tracheostomiekanüle angebrachten offenbarungsgemäßen Schildes mit einem eckig ausgeführten brückenartigen Befestigungselement.
- Figur 2: zeigt den Vergleich eines herkömmlichen Schildes aus dem Stand der Technik, der Befestigungsösen aufweist, die in Form von durchgängigen Ausnehmungen in der Schildfläche vorgesehen sind (Abbildung 2a), mit einem offenbarungsgemäßen Schild, bei dem die Ösen durch ein auf der proximalen Fläche des Schildes angeordnetes brückenartiges Befestigungselement vorgesehen sind (Abbildung 2b).
- Figur 3: zeigt eine Ausführungsform eines an einem Kanülenrohr einer Tracheostomiekanüle angebrachten offenbarungsgemäßen Schildes mit einem abgerundet ausgeführten brückenartigen Befestigungselement.
- Figur 4: zeigt eine Ausführungsform eines offenbarungsgemäßen Schildes mit Anhebeelementen für das Halteband.
- Figur 5: zeigt eine Ausführungsform eines offenbarungsgemäßen Schildes mit nutförmigen Ausnehmungen in der Schildfläche, durch die das Halteband geführt werden kann.
- Figur 6: zeigt eine Ausführungsform eines offenbarungsgemäßen Schildes, bei der unter den brückenartigen Befestigungselementen durchgängige Ausnehmungen in der Schildfläche vorgesehen sind, durch die das Halteband geführt werden kann.
- Figur 7: zeigt eine alternative Ausführungsform eines offenbarungsgemäßen Schildes, bei der unter den brückenartigen Befestigungselementen im Vergleich zu der Ausführungsform von Figur 6 etwas anders ausgerichtete durchgängige Ausnehmungen vorgesehen sind.
- Figur 8: zeigt eine Ausführungsform eines offenbarungsgemäßen Schildes mit einer Kombination von nutförmigen Ausnehmungen und durchgängigen Ausnehmungen im Bereich der brückenartigen Befestigungselemente.
- Figur 9: zeigt eine Ausführungsform eines offenbarungsgemäßen Schildes, bei der das Material des Schildes abschnittsweise aus einem härteren Kunststoffmaterial und abschnittsweise aus einem weicheren Kunststoffmaterial gebildet ist.
- Figur 10: zeigt im Vergleich zu Figur 9 eine alternative Ausführungsform eines offenbarungsgemäßen Schildes mit Bereichen aus unterschiedlich harten Kunststoffmaterialien.
- Figur 11: zeigt eine Ausführungsform eines offenbarungsgemäßen Schildes mit gewölbten Schildflächen.
- Figur 12: zeigt eine Tracheostomiekanüle mit einem daran kardanisch gelagerten offenbarungsgemäßen Schild.
- Figur 13: zeigt eine Ausführungsform eines offenbarungsgemäßen Schildes mit Polsterelement, wobei Schild und Polsterelement einmal separat dargestellt sind (oben) und einmal im zusammengesetzten Zustand dargestellt sind (unten).
- Figur 14: zeigt eine besondere Variante des Polsterelements eines offenbarungsgemäßen Schildes mit einem an dem Schild vorgesehenen Schnappverschluss einmal in Draufsicht von der Seite (links) und einmal ein Detail des Polsterelements im geschnittenen Zustand.
- Figur 15: zeigt eine Ausführungsform eines offenbarungsgemäßen Schildes mit einem Polsterelement, das auf der Unterseite ein konisches Anschlussstück aufweist.

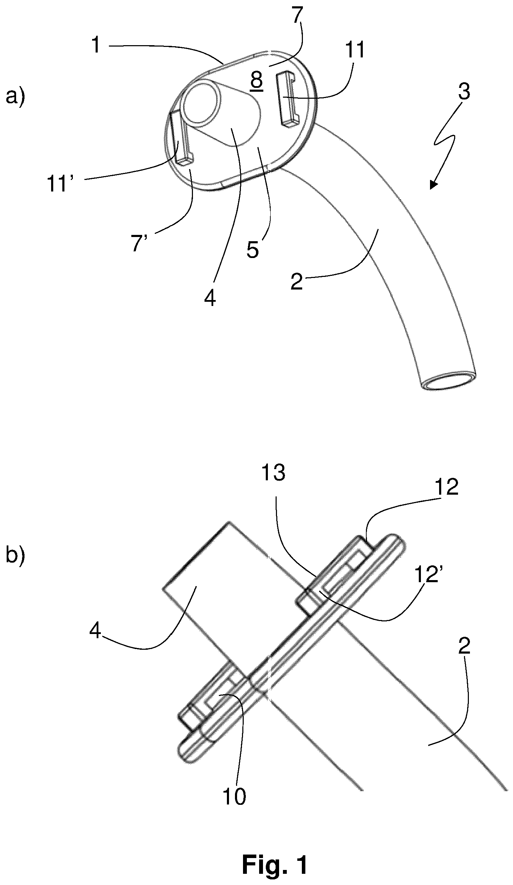

In Figur 1a) ist eine perspektivische Draufsicht auf eine Tracheostomiekanüle 3 mit einem daran angeordneten offenbarungsgemäßen Schild 1 dargestellt. Am proximalen Ende des Kanülenrohrs 2 befindet sich ein Konnektor 4, der durch eine entsprechende Ausnehmung im zentralen Bereich 5 des Schildes 1 geführt ist. In dieser Ansicht ist die proximale Fläche 8 des Schildes 1 zu sehen, die sich über den zentralen Bereich 5 und die beiden Seitenteile 7, 7' erstreckt, die sich an zwei gegenüberliegenden Seiten des Zentralbereichs 5 anschließen und sich von dort in radialer Richtung bis zum jeweiligen Ende des Seitenteiles 7, 7' erstrecken. Im Endbereich der Seitenteile 7, 7' ist jeweils ein brückenartiges Befestigungselement 11, 11' angeordnet, das zwei auf der proximalen Fläche 8 des Schildes aufstehende Pfeilerabschnitte 12, 12' und einen sich zwischen den beiden Pfeilerabschnitten 12, 12' erstreckenden Überbrückungsabschnitt 13 aufweist (vgl. Figur 1b). Die Pfeilerabschnitte 12, 12' sind in einem Abstand vom Ende des jeweiligen Seitenteiles angeordnet, sodass die brückenartigen Befestigungselemente 11, 11' insgesamt in einem Abstand vom jeweiligen Ende des Seitenteiles 7, 7' angeordnet sind, um dort jeweils eine Öse 10, 10' zu bilden. Diese Öse 10, 10' erstreckt sich über den Bereich, der sich über dem von dem brückenartigen Befestigungselement 11, 11' überspannten Bereich über der proximalen Fläche 8 des Schildes 1 erstreckt.

Bei der hier dargestellten Ausführungsform ist das brückenartige Befestigungselement 11, 11' eckig ausgeführt, wobei die Kanten und Ecken des brückenartigen Befestigungselementes 11, 11' abgerundet ausgeführt sind, um die potentielle Verletzungsgefahr an diesen Ecken und Kanten so gering wie möglich zu halten.

Figur 1b) zeigt den proximalen Bereich der in Figur 1a) gezeigten Tracheostomiekanüle 3 stark vergrößert und in einer anderen Perspektive, um die Pfeilerabschnitte 12, 12' und den Überbrückungsabschnitt 13 der Befestigungselemente 11, 11' noch einmal deutlicher darstellen zu können. Darüber hinaus ist in dieser Darstellung auch die durch das brückenartige Element 11, 11' im Zusammenspiel mit der proximalen Fläche 8 des Schildes 1 gebildete Öse 10, 10' besser zu erkennen.

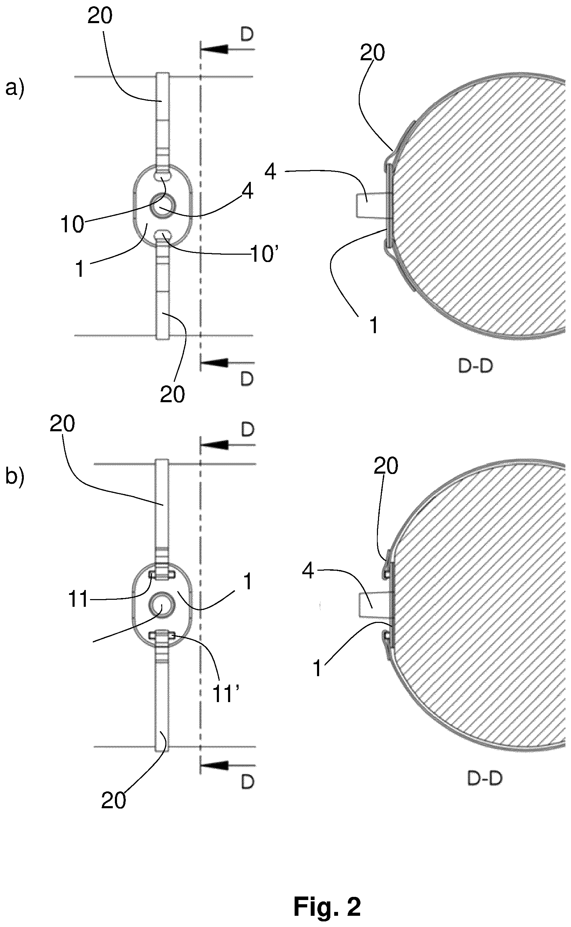

In Figur 2a) ist ein herkömmliches Schild 1, wie es aus dem Stand der Technik bekannt ist, dargestellt. Bei diesem herkömmlichen Schild 1 werden die Ösen 10, 10 Strich für das Halteband 20 in Form von durchgängigen Ausnehmungen in dem Schild 1 bereitgestellt. In der linken Abbildung von Figur 2a) ist eine Draufsicht auf das herkömmliche Schild 1 mit daran angeordnetem Halteband 20 gezeigt. Gezeigt ist hier der Zustand eines am Hals eines Patienten mithilfe des Haltebandes 20 fixierten Schildes 1.

In der rechten Abbildung von Figur 2a) ist der Schnitt entlang der Linie D-D dargestellt, um zu zeigen, dass bei dieser herkömmlichen Lösung das Halteband 20 teilweise unter dem Schild 1, d.h. zwischen Schild 1 und Hals des Patienten (schraffierter Bereich), geführt ist.

In Figur 2b) ist ein offenbarungsgemäßen Schild mit Befestigungselementen 11, 11' und daran angeordnetem Halteband 20 ebenfalls im am Patientenhals angebrachten Zustand dargestellt. Auf der linken Seite von Figur 2b) ist eine Draufsicht von oben auf den Schild 1 zu sehen, während in der rechten Abbildung ein Schnitt entlang der Linie D-D dargestellt ist. Anhand dieser Darstellung ist gut zu erkennen, dass bei einem Schild gemäß der vorliegenden Offenbarung das Halteband 20 nicht zwischen Schild und Hals des Patienten geführt wird, sodass es hier auch nicht zu den unerwünschten Reibeffekten kommen kann.

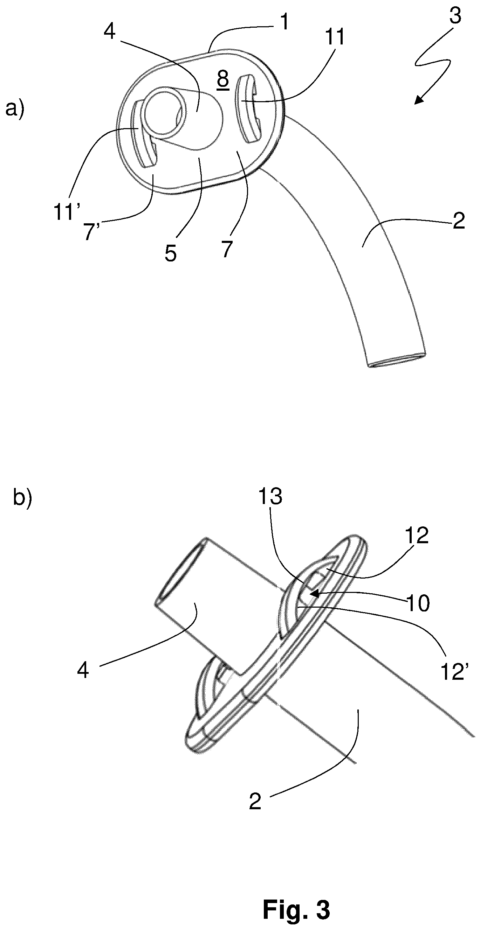

In Figur 3a) ist eine alternative Ausführungsform eines offenbarungsgemäßen Schildes, das am Kanülenrohr 2 einer Tracheostomiekanüle 3 im Bereich des Konnektors 4 angebracht ist, dargestellt. Bei der hier dargestellten Ausführungsform sind die jeweils im Abstand von den Enden der Seitenteile 7, 7' angeordneten brückenartigen Befestigungselemente 11, 11' abgerundet ausgeführt, wobei die Pfeilerabschnitte 12, 12' in den Überbrückungsabschnitt 13 fließend übergehen.

Figur 3b) zeigt das proximale Ende der in Figur 3a) dargestellten Ausführungsform Offenbarung stark vergrößert und in einer anderen Perspektive, in der die Ösen 10, 10', die sich dadurch ausbilden, dass die brückenartigen Befestigungselemente 11, 11' auf der proximalen Fläche 8 des offenbarungsgemäßen Schildes 1 angeordnet sind, ausgebildet werden.

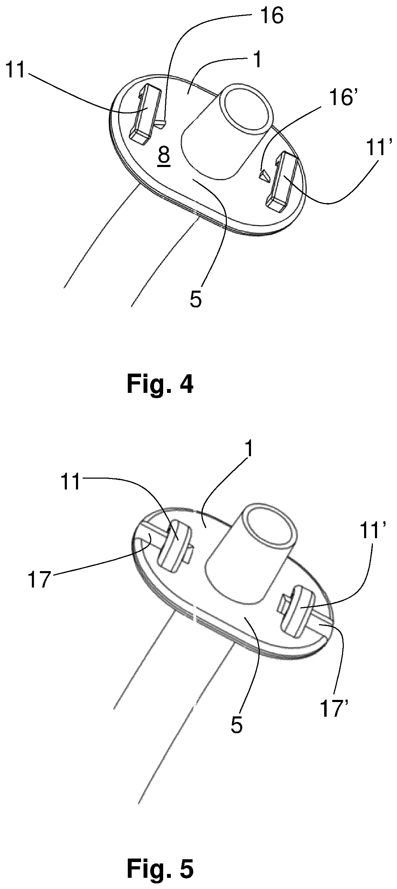

Figur 4 zeigt eine Ausführungsform eines offenbarungsgemäßen Schildes, bei der auf der proximalen Fläche 8 zwischen den brückenartigen Befestigungselementen 11, 11' und dem zentralen Bereich 5 Anhebeelemente 16, 16' vorgesehen sind. Diese Anhebeelemente 16, 16'weisen eine in Richtung des zentralen Bereichs 5 ansteigende Anhebefläche auf, die auf der Seite zur Öse hin mit der proximalen Fläche 8 des Schildes 1 im Wesentlichen bündig abschließt. Durch das in Richtung des zentralen Bereichs ansteigende Ende der Anhebefläche wird das Ende eines aus der Richtung des Seitenteilendes in Richtung des zentralen Bereichs 5 durch die Öse geführten Haltebandes (nicht dargestellt) von der proximalen Fläche des Seitenteils leicht abgehoben, sodass man dieses Ende leicht ergreifen kann.

In Figur 5 ist eine Ausführungsform eines offenbarungsgemäßen Schildes 1 dargestellt, bei dem die brückenartigen Befestigungselemente 11, 11' abgerundet ausgeführt sind, wobei die Gesamthöhe der brückenartigen Befestigungselemente 11, 11' hier relativ gering gehalten werden kann, da unter den brückenartigen Befestigungselementen 11, 11' nutartige Ausnehmungen vorgesehen sind, die sich bis zum Ende des Seitenteils durchgehend erstrecken. Auf diese Weise wird eine Öse mit einer hinreichend großen lichten Höhe erreicht, sodass das Halteband problemlos hindurchgeführt werden kann. In Richtung des zentralen Bereiches 5 steigt der Nutboden bei der hier dargestellten Ausführungsform bis auf das Niveau der proximalen Fläche 8 an. Ein in diese Richtung eingeführtes Halteband wird somit in proximale Richtung geführt und kann damit vom Anwender leicht ergriffen werden.

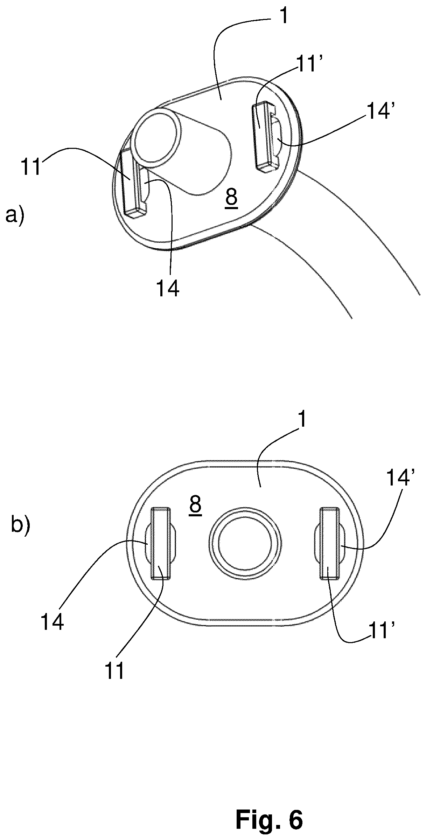

In Figur 6 ist eine Ausführungsform eines offenbarungsgemäßen Schildes 1 dargestellt, bei der unter den brückenartigen Befestigungselementen 11, 11' von der proximalen Fläche bis zur distalen Fläche des Schildes durchgängige Ausnehmungen 14, 14' vorgesehen sind. Bei der hier dargestellten Ausführungsform sind die durchgängigen Ausnehmungen 14, 14' in Form von Langlöchern vorgesehen, die sich von der einen Pfeilerabschnittinnenseite eines brückenartigen Befestigungselementes 11, 11' zur Innenseite des anderen Pfeilerabschnitts des gleichen brückenartigen Befestigungselements 11, 11' erstrecken. Diese durchgängigen Ausnehmungen 14, 14' bieten die Möglichkeit, das Kanülenband je nach den gegebenen Umständen alternativ durch die durchgängigen Ausnehmungen einzufädeln, sofern dies vom Anwender gewünscht ist. In Figur 6a) ist diese spezielle Ausführungsform in einer perspektivischen Ansicht dargestellt. In Figur 6b) ist dieselbe Ausführungsform noch einmal in Draufsicht von oben auf die proximale Fläche 8 des Schildes 1 dargestellt.

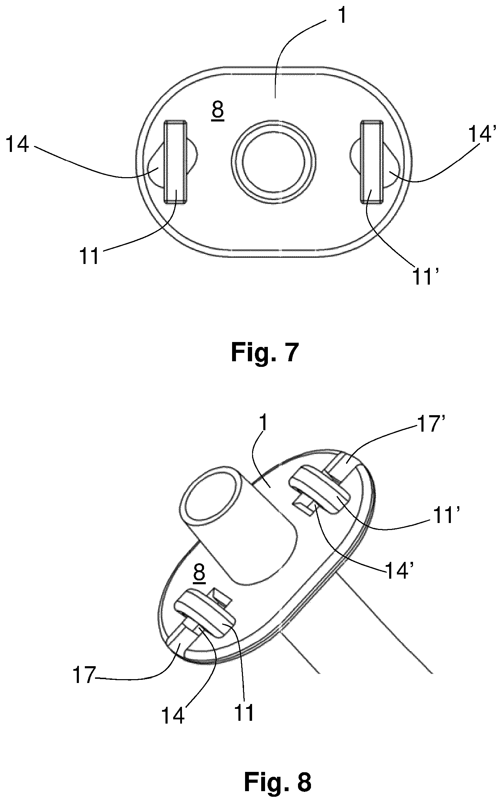

In Figur 7 ist eine alternative Ausführungsform eines offenbarungsgemäßen Schildes 1 mit unter den brückenartigen Befestigungselementen 11, 11' angeordneten durchgängigen Ausnehmungen 14, 14' dargestellt, wobei die durchgängige Ausnehmung länglich ist und wobei die Längsachse der Ausnehmung im Unterschied zu der in Figur 6 dargestellten Ausführungsform auf einer Linie liegt, welche Linie, auf der die Pfeilerabschnitte des Befestigungselementes auf der proximalen Fläche aufstehen, in einem Winkel von ca 45° schneidet.

In Figur 8 ist eine alternative Ausführungsform des offenbarungsgemäßen Schildes 1 mit unter den brückenartigen Befestigungselementen 11, 11' angeordneten durchgängigen Ausnehmungen 14, 14' dargestellt, wobei bei dieser Ausführungsform zusätzlich noch nutartige Ausnehmungen 17, 17' vorgesehen sind, die sich an die durchgängigen Ausnehmungen 14, 14' in Richtung der seitlichen Enden des Schildes 1 durchgängig erstrecken.

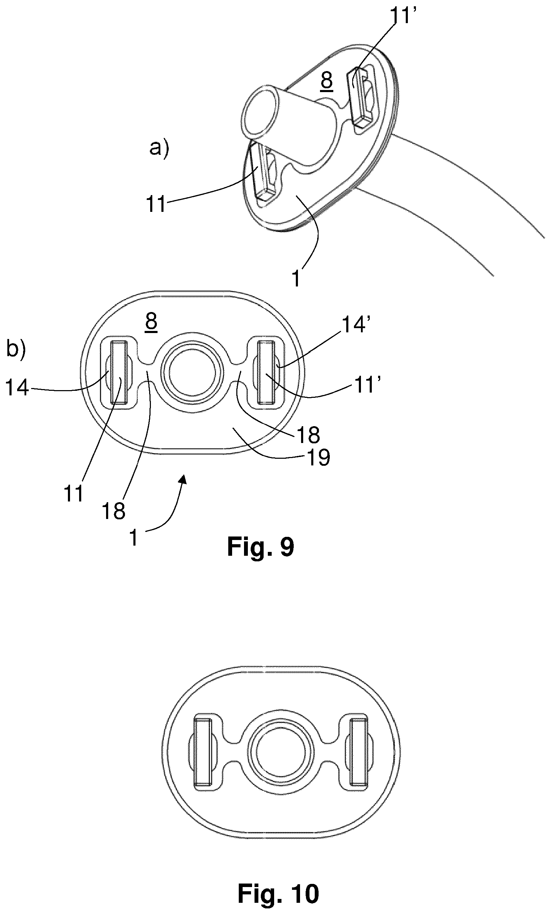

Die Figuren 9 und 10 zeigen alternative Ausführungsformen des offenbarungsgemäßen Schildes. Bei den hier dargestellten Ausführungsformen sind zusätzlich zu den brückenartigen Befestigungselementen 11, 11' durchgängige Ausnehmungen 14, 14' vorgesehen. Besonders bei den hier dargestellten Ausführungsformen ist, dass das Schildmaterial in manchen Bereichen des Schildes 1 aus einem härteren Kunststoffmaterial besteht und in anderen Bereichen aus einem weicheren Kunststoffmaterial. Die Bereiche mit härterem Kunststoffmaterial 18 erstrecken sich wenigstens teilweise oder vollständig um die brückenartigen Befestigungselemente 11, 11', die durchgängigen Ausnehmungen 14, 14' und die im zentralen Bereich des Schildes angeordnete durchgängige Ausnehmung zum Hindurchführen einer Kanüle oder eines Konnektors. Dies dient dazu, in diesen Bereichen besonders hohe Stabilität bereitzustellen, da diese Bereiche für die Fixierung des Haltebandes und für die Stabilisierung der Kanüle besonders wichtig sind. Die anderen Bereiche, die aus einem weicheren Kunststoffmaterial 19 bestehen, sorgen dagegen für einen besseren Tragekomfort und eine geringere mechanische Beanspruchung des Halses des Patienten.

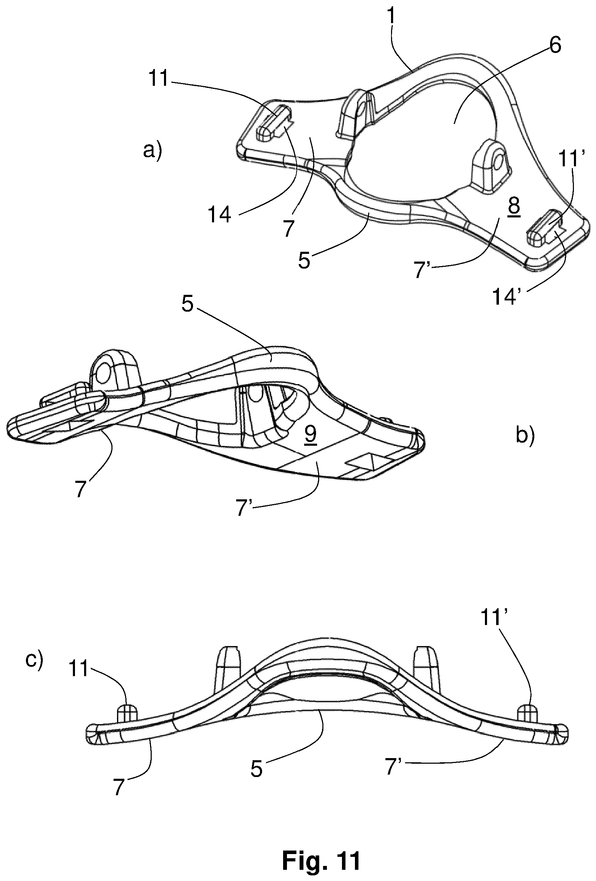

In Figur 11 werden verschiedene Ansichten einer besonderen Ausführungsform eines offenbarungsgemäßen Schildes 1 dargestellt, bei der sowohl die proximale Fläche 8 des Schildes als auch die distale Fläche 9 des Schildes in besonderer Weise gewölbt ausgeführt sind. Dabei ist der zentrale Bereich 5 so in proximale Richtung aufgewölbt, dass er am Patientenhals nicht zum Aufliegen kommt, wenn der Schild 1 mit einem Halteband am Hals des Patienten befestigt wird. Auf diese Weise wird in diesem Bereich unnötige Reibung am Patientenhals vermieden.

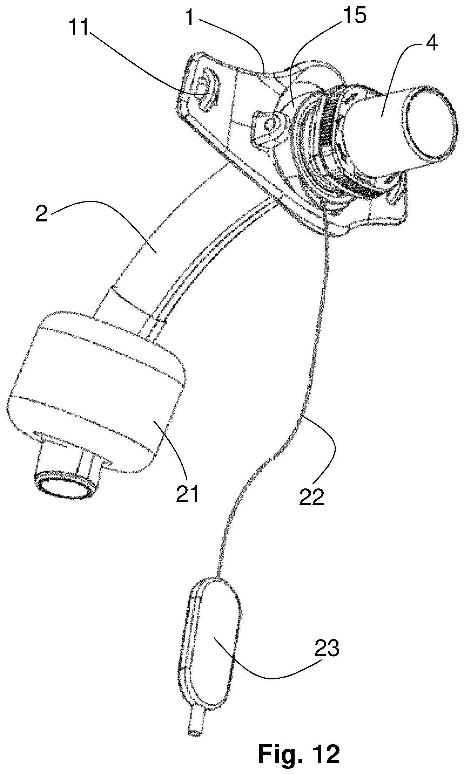

In Figur 12 ist eine Ausführungsform eines offenbarungsgemäßen Schildes 1 dargestellt, der über eine kardanische Lagerung 15 an der Kanüle 2 angeordnet ist. Die durch die kardanische Lagerung 15 erreichte zusätzliche Beweglichkeit des Schildes 1 ermöglicht die optimale individuelle Anpassung an die gegebene Halsanatomie des jeweiligen Patienten.

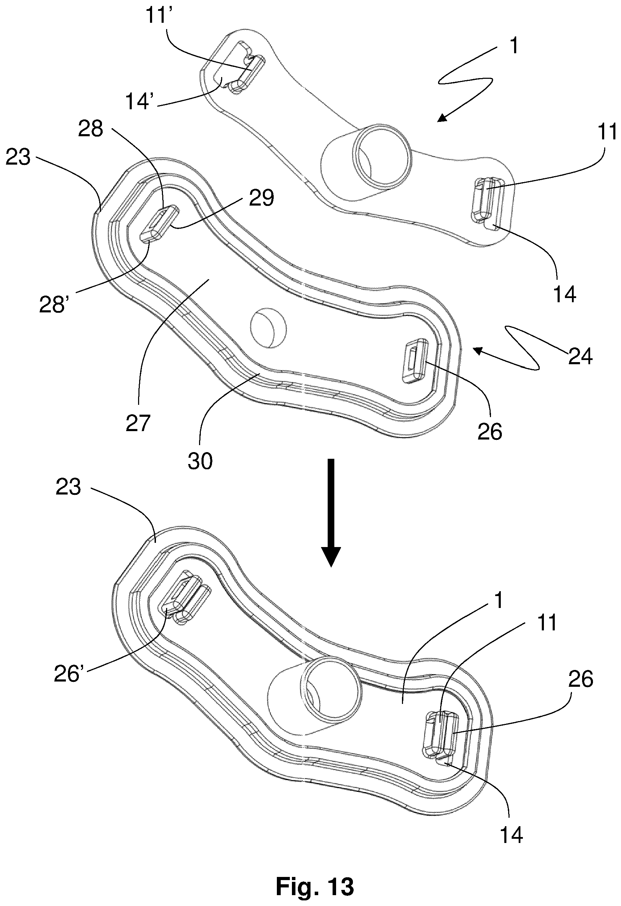

Figur 13 zeigt eine Ausführungsform eines offenbarungsgemäßen Schildes mit einem Polsterelement. Im oberen Teil sind der Schild 1 und das Polsterelement 24 separat, d.h. im nicht zusammengesetzten Zustand, dargestellt. Unterhalb des in der Figur gezeigten Pfeiles ist dann der zusammengesetzte Zustand dargestellt, bei dem der Schild 1 auf dem Polsterelement 24 angeordnet ist.

Wie insbesondere in der obigen Darstellung zu erkennen ist, sind auf der proximalen Fläche des Schildes 1 in einem Abstand zum Ende der Seitenteile die Befestigungselemente 11, 11' angeordnet. Außerdem befinden sich dort die durchgängigen Ausnehmungen 14, 14'. Des Weiteren ist in der obigen Darstellung das Polsterelement 24 mit den Befestigungselementen 26, 26' gut zu erkennen. Wie bei dem auf dem Schild 1 angeordneten Befestigungselementen 11, 11' bestehen auch die Befestigungselemente 26, 26' auf dem Polsterelement 24 aus Pfeilerabschnitten 28, 28' und einem dazwischenliegenden Überbrückungsabschnitt 29, sodass ein brückenartiges Befestigungselement entsteht, das eine Öse bildet, durch welche ein Halteband geführt werden kann.

Das Polsterelement 24 ist abgesehen von den auf der proximalen Fläche 27 aufstehenden Befestigungselementen 26, 26' im Wesentlichen flächig ausgestaltet. Bei der hier dargestellten Ausführungsform ist allerdings noch ein vollständig umlaufender Ringwulst 30 vorgesehen, der zum Halten des Schildes 1 im Polsterelement 24 vorgesehen ist. Offenbarungsgemäß besteht das im wesentlichen flächige Polsterelement aus einem weichen, elastisch verformbaren Material, und die geometrische Form ist so gewählt, dass der Außenumfang des flächigen Polsterelementes 24 über den Außenumfang der distalen Fläche des Schildes 1 allseits um wenigstens 1 mm übersteht.

In der zusammengesetzten Darstellung unterhalb des Falles in Pfeiles 13 ist gut zu erkennen, dass die Befestigungselemente 26, 26' des Polsterelements 24 so angeordnet sind, dass sie durch die in den Seitenteilen des Schildes 1 angeordneten durchgängigen Ausnehmungen 14, 14' ragen und dabei so neben den brückenartigen Befestigungselementen 11, 11' des Schildes 1 angeordnet sind, dass die Ösen des Polsterelementes 24 mit den Ösen des Schildes 1 im Wesentlichen zur Deckung kommen. Ein durch die Ösen der Befestigungselemente 11, 11' des Schildes 1 geführtes Halteband kann somit gleich auch durch die von den Befestigungselementen 26, 26' des Polsterelementes 24 gebildeten Ösen geführt werden. Schild 1 und Polsterelement 24 werden somit durch dasselbe Halteband sowohl zueinander als auch am Hals des Patienten in Position gehalten.

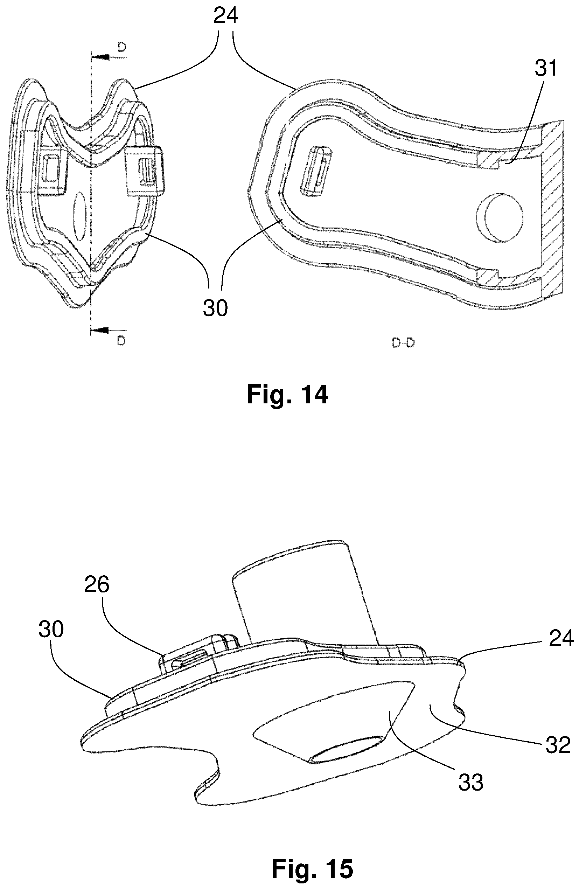

In Figur 14 ist das Polsterelement 24 der in Figur 13 dargestellten Ausführungsform des offenbarungsgemäßen Schildes 1 mit Polsterelement 24 einmal in einer Draufsicht von der Seite gezeigt (linke Seite von Figur 14). Auf der rechten Seite von Figur 14 befindet sich eine geschnittene Detailansicht des Polsterelementes 24 entlang der Schnittebene D-D, wie sie in der Darstellung auf der linken Seite von Figur 14 eingezeichnet ist. Insbesondere im geschnittenen Detail ist der Ringwulst 30, der auf der proximalen Fläche des Polsterelementes 24 vorgesehen ist, gut zu erkennen. Ebenfalls zu erkennen ist hier die Hinterschneidung 31, die so gestaltet ist, dass ein auf der Polsterung 24 montierter Schild in die Hinterschneidung 31 des Ringwulstes 30 rutschen kann, da auch der Ringwulst 30, wie die gesamte Polsterung 24, aus einem weichen, elastischen Material besteht. Der Schild wird damit auf der Polsterung 24 formschlüssig gehalten (vgl. untere Darstellung von Figur 13).

In Figur 15 ist eine besondere Ausführungsform eines Polsterelementes 24 für den offenbarungsgemäßen Schild dargestellt. Bei der hier dargestellten Ausführungsform befindet sich auf der distalen Fläche 32 des Polsterelementes 24 ein konisches Anschlussstück 33, das insbesondere bei Patienten mit relativ großem Stoma dazu dienen kann, für eine bessere Abdichtung zu sorgen, damit möglichst wenig Sprechluft an dieser Stelle entweichen kann. Je nachdem wie groß das Stoma des jeweiligen Patienten ist, kann ein Polsterelement 24 verwendet werden, das einen konischen Abschnitt mit entsprechend geeignetem Außendurchmesser, geeigneter Höhe und geeigneter Steigung aufweist.

### Bezugszeichenliste

- 1: Schild
- 2: Kanülenrohr
- 3: Tracheostomiekanüle
- 4: Konnektor
- 5: zentraler Bereich
- 6: durchgängige Ausnehmung
- 7, 7': Seitenteil
- 8: proximale Fläche
- 9: distale Fläche
- 10, 10': Öse
- 11, 11': Befestigungselement
- 12, 12': Pfeilerabschnitte
- 13: Überbrückungsabschnitt
- 14, 14': durchgängige Ausnehmung
- 15: kardanische Lagerung
- 16, 16': Anhebeelement
- 17, 17': nutförmige Ausnehmung
- 18: Bereich aus hartem Kunststoffmaterial
- 19: Bereich aus weichem Kunststoffmaterial
- 20: Halteband
- 21: Cuff
- 22: Cuff-Zuleitung
- 23: Füllballon
- 24: Polsterelement
- 25, 25': Öse des Polsterelements
- 26, 26': Befestigungselement des Polsterelements
- 27: proximale Fläche des Polsterelements
- 28, 28': Pfeilerabschnitte
- 29: Überbrückungsabschnitt
- 30: Ringwulst
- 31: Hinterschneidung
- 32: distale Fläche des Polsterelements
- 33: konischer Abschnitt

## Patentansprüche

1. Schild (1) zur Anbringung an einem Kanülenrohr (2) einer Tracheostomiekanüle (3) oder an einem an dem Kanülenrohr (2) proximal angeordneten ringförmigen Konnektor (4), wobei der Schild (1)
- einen zentralen Bereich (5) aufweist, der eine durchgängige Ausnehmung (6) zum Hindurchführen des Kanülenrohrs (2) und/oder des Konnektors (4) aufweist, und
- zwei Seitenteile (7, 7'), die sich an zwei gegenüberliegenden Seiten des zentralen Bereichs (5) anschließen und sich von dort in radialer Richtung bis zum jeweiligen Ende des Seitenteils (7, 7') erstrecken,
wobei der Schild (1) eine proximale Seite mit einer proximalen Fläche (8) aufweist, die im Gebrauch vom Hals des Patienten weg weist, und eine distale Seite mit einer distalen Fläche (9) aufweist, die im Gebrauch zum Hals des Patienten hin weist, wobei die beiden Seitenteile jeweils eine Öse (10, 10') zum Befestigen eines Haltebandes aufweisen,
, wobei
die jeweils eine Öse (10, 10') von einem brückenartigen Befestigungselement (11, 11') gebildet wird, das zwei auf der proximalen Fläche (8) des Schildes (1) angeordnete Pfeilerabschnitte (12, 12') und einen sich zwischen den beiden Pfeilerabschnitten (12, 12') erstreckenden Überbrückungsabschnitt (13) aufweist, wobei beide Pfeilerabschnitte (12, 12') in einem Abstand vom Ende des jeweiligen Seitenteils (7, 7') angeordnet sind, **dadurch gekennzeichnet, dass** beide Seitenteile (7, 7') in dem von dem Befestigungselement (11, 11') überspannten Bereich eine von der proximalen Fläche (8) bis zur distalen Fläche (9) durchgängige Ausnehmung (14, 14') aufweisen, die optional in Richtung des Endes des Seitenteils (7, 7') um circa 1 bis 3 mm über den Bereich hinausgeht, der von dem brückenartigen Befestigungselement (11, 11') überspannt wird.

2. Schild gemäß Anspruch 1, **dadurch gekennzeichnet, dass** der Abstand zwischen den Innenseiten der Pfeilerabschnitte eines brückenartigen Befestigungselements im Bereich von 3 bis 12 mm liegt und/oder die lichte Höhe zwischen der proximalen Fläche des Schildes und der Innenseite des Überbrückungsabschnitts im Bereich von 2 bis 5 mm liegt.

3. Schild gemäß einem der Ansprüche 1 oder 2, **dadurch gekennzeichnet, dass** der Abstand der Pfeilerabschnitte vom Ende des jeweiligen Seitenteils wenigstens 4 mm, noch bevorzugter wenigstens 6 mm beträgt.

4. Schild gemäß einem der vorangehenden Ansprüche, **dadurch gekennzeichnet, dass** die beiden Pfeilerabschnitte des jeweiligen Befestigungselements und der sich zwischen den beiden Pfeilerabschnitten erstreckenden Überbrückungsabschnitt einen rechteckigen, einen abgerundet rechteckigen, einen ovalen oder kreisrunden Querschnitt aufweisen.

5. Schild gemäß einem der vorangehenden Ansprüche, **dadurch gekennzeichnet, dass** die beiden Pfeilerabschnitte des jeweiligen Befestigungselements mit dem sich zwischen den beiden Pfeilerabschnitten erstreckenden Überbrückungsabschnitt jeweils einen Winkel im Bereich von 90° bis 135° bilden oder die beiden Pfeilerabschnitte des jeweiligen Befestigungselements mit dem sich zwischen den beiden Pfeilerabschnitten erstreckenden Überbrückungsabschnitt einen Rundbogen beschreiben.

6. Schild gemäß einem der vorangehenden Ansprüche, **dadurch gekennzeichnet, dass** die beiden Pfeilerabschnitte des jeweiligen Befestigungselements in einem rechten Winkel auf der proximalen Fläche aufstehen oder in einem Winkel, der um +/- 45° vom rechten Winkel abweicht.

7. Schild gemäß einem der vorangehenden Ansprüche, **dadurch gekennzeichnet, dass** die distale Fläche der Seitenteile im Wesentlichen parallel zu der proximalen Fläche des jeweiligen Seitenteils verläuft.

8. Schild gemäß einem der vorangehenden Ansprüche, **dadurch gekennzeichnet, dass** die Seitenfläche, die sich zwischen der distalen Fläche und der proximalen Fläche eines Seitenteils erstreckt, am Ende des Seitenteils über die Breite des Schildes auf einer bogenförmigen Bahn verläuft und/oder ohne Ausbildung einer Kante fließend in die distale Fläche und wahlweise auch in die proximale Fläche des jeweiligen Seitenteils übergeht.

9. Schild gemäß einem der vorangehenden Ansprüche, **dadurch gekennzeichnet, dass** der Schild aus einem weichen, elastisch verformbaren Material oder aus einem harten, formstabilen Material oder zum einen Teil aus einem weichen, elastisch verformbaren Material und zum anderen Teil aus einem harten, formstabilen Material besteht.

10. Schild gemäß einem der vorangehenden Ansprüche, **dadurch gekennzeichnet, dass** die Seitenteile des Schildes flächig ausgebildet sind und vorzugsweise eine Materialstärke im Bereich von 1,5 mm bis 4 mm aufweisen.

11. Schild gemäß einem der vorangehenden Ansprüche, **dadurch gekennzeichnet, dass** die distale Fläche im zentralen Bereich einen in Längsrichtung des Schildes in Richtung proximal konkav gekrümmten Abschnitt und/oder einen senkrecht zur Längsrichtung des Schildes in Richtung proximal konkav gekrümmten Abschnitt aufweist.

12. Schild gemäß einem der vorangehenden Ansprüche, **dadurch gekennzeichnet, dass** der Schild wenigstens eine der folgenden Einrichtung aufweist:
- im zentralen Bereich eine Aufhängeinrichtung zur kardanischen Lagerung (15) am Kanülenrohr oder an einem Konnektor einer Tracheostomiekanüle,
- auf der proximalen Fläche zwischen der Öse und dem zentralen Bereich ein Anhebeelement (16, 16') mit einer Anhebefläche, die auf der Seite zur Öse hin mit der proximalen Fläche im Wesentlichen bündig abschließt und in Richtung zentraler Bereich um 1 bis 5 mm oder 1 bis 3 mm ansteigt,
- in der proximalen Fläche jeweils eine muldenförmige oder nutartige Ausnehmung (17, 17') in dem Bereich der von den brückenartigen Befestigungselementen überspannt wird und optional darüber hinaus,
- in den Seitenteilen von der proximalen Fläche bis zur distalen Fläche durchgängige Ausnehmungen (14, 14'), die sich wenigstens über den Bereich erstrecken, der sich von den brückenartigen Befestigungselementen überspannt wird, oder die sich wenigstens über den Bereich erstrecken, der sich neben dem von den brückenartigen Befestigungselementen überspannten Bereich erstreckt,
- an der distalen Fläches des Schildes anliegend ein separates flächiges Polsterelement (24) aus einem weichen, elastisch verformbaren Material, wobei der Außenumfang des flächigen Polsterelements über den Außenumfang der distalen Fläche des Schildes allseits um wenigstens 1 mm übersteht.

13. Schild gemäß einem der vorangehenden Ansprüche, **dadurch gekennzeichnet, dass** in den Seitenteilen von der proximalen Fläche bis zur distalen Fläche durchgängige Ausnehmungen (14, 14') vorgesehen sind, die sich wenigstens über den Bereich erstrecken, der sich neben dem von den brückenartigen Befestigungselementen überspannten Bereich erstreckt, wobei der Schild an dessen distaler Fläche anliegend ein separates flächiges Polsterelement (24) aus einem weichen, elastisch verformbaren Material aufweist, wobei der Außenumfang des flächigen Polsterelements über den Außenumfang der distalen Fläche des Schildes allseits um wenigstens 1 mm übersteht, wobei das separate Polsterelement (24) zwei Ösen (25, 25') zum Befestigen eines Haltebandes aufweist, wobei die jeweils eine Öse (25, 25') von einem brückenartigen Befestigungselement (26, 26') gebildet wird, das zwei auf der proximalen Fläche (27) des Polsterelements (24) angeordnete Pfeilerabschnitte (28, 28') und einen sich zwischen den beiden Pfeilerabschnitten (28, 28') erstreckenden Überbrückungsabschnitt (29) aufweist, wobei die beiden brückenartigen Befestigungselemente (26, 26') des Polsterelements (24) so vorgesehen sind, dass sie durch in den Seitenteilen des Schildes (1) angeordnete durchgängige Ausnehmung (14, 14') ragen und dadurch so neben den brückenartigen Befestigungselementen (11, 11') des Schildes angeordnet sind, dass die Ösen (25, 25') des Polsterelements (24) mit den Ösen (10, 10') des Schildes (1) im Wesentlichen zur Deckung kommen.

14. Tracheostomiekanüle zur Einbringung in ein Tracheostoma mit einem Kanülenrohr und einem Schild gemäß einem der Ansprüche 1 bis 13.

## Claims

1. Shield (1) for mounting on a cannula tube (2) of a tracheostomy cannula (3) or on an annular connector (4) arranged proximally on the cannula tube (2), wherein the shield (1) has
- a central region (5) comprising a continuous recess (6) through which the cannula tube (2) and/or the connector (4) is guided, and
- two side parts (7, 7') which adjoin two opposite sides of the central region (5) and extend radially from there to the respective end of the side part (7, 7'), wherein the shield (1) has a proximal side with a proximal surface (8), which faces away from the patient's neck during use, and has a distal side with a distal surface (9), which faces towards the patient's neck during use, wherein the two side parts each have an eyelet (10, 10') for fastening a retaining strap,
wherein
the respective eyelet (10, 10') is formed by a bridge-like fastening element (11, 11'), which has two pillar portions (12, 12') arranged on the proximal surface (8) of the shield (1)
and a bridging portion (13) which extends between the two pillar portions (12, 12'), wherein both pillar portions (12, 12') are arranged at a distance from the end of the respective side part (7, 7'), **characterized in that** both side parts (7, 7'), in the region spanned by the fastening element (11, 11'), have a recess (14, 14') which is continuous from the proximal surface (8) to the distal surface (9) and which optionally protrudes in the direction of the end of the side part (7, 7') by about 1 to 3 mm beyond the region that is spanned by the bridge-like fastening element (11, 11').

2. Shield according to Claim 1, **characterized in that** the distance between the inner sides of the pillar portions of a bridge-like fastening element is in the range of 3 to 12 mm, and/or the clear height between the proximal surface of the shield and the inner side of the bridging portion is in the range of 2 to 5 mm.

3. Shield according to either of Claims 1 and 2, **characterized in that** the distance of the pillar portions from the end of the respective side part is at least 4 mm, more preferably at least 6 mm.

4. Shield according to any one of the preceding claims, **characterized in that** the two pillar portions of the respective fastening element and the bridging portion extending between the two pillar portions have a rectangular, a roundedly rectangular, an oval or circular cross section.

5. Shield according to any one of the preceding claims, **characterized in that** the two pillar portions of the respective fastening element form, with the bridging portion extending between the two pillar portions, in each case an angle in the range of 90° to 135°, or the two pillar portions of the respective fastening element, together with the bridging portion extending between the two pillar portions, describe a circular arc.

6. Shield according to any one of the preceding claims, **characterized in that** the two pillar portions of the respective fastening element stand at a right angle on the proximal surface, or at an angle that deviates by +/-45° from the right angle.

7. Shield according to any one of the preceding claims, **characterized in that** the distal surface of the side parts runs substantially parallel to the proximal surface of the respective side part.

8. Shield according to any one of the preceding claims, **characterized in that** the side surface which extends between the distal surface and the proximal surface of a side part runs, at the end of the side part, over the width of the shield on an arc-shaped path and/or, without forming an edge, transitions seamlessly into the distal surface and optionally also into the proximal surface of the respective side part.

9. Shield according to any one of the preceding claims, **characterized in that** the shield is made of a soft, elastically deformable material or of a hard, dimensionally stable material or partly of a soft, elastically deformable material and partly of a hard, dimensionally stable material.

10. Shield according to any one of the preceding claims, **characterized in that** the side parts of the shield are designed to be flat and preferably have a material thickness in the range of 1.5 mm to 4 mm.

11. Shield according to any one of the preceding claims, **characterized in that** the distal surface in the central region has a proximally concavely curved portion in the longitudinal direction of the shield and/or a proximally concavely curved portion perpendicular to the longitudinal direction of the shield.

12. Shield according to any one of the preceding claims, **characterized in that** the shield has at least one of the following:
- in the central region, a suspension device (15) for cardanic mounting on the cannula tube or on a connector of a tracheostomy cannula,
- on the proximal surface between the eyelet and the central region, a lifting element (16, 16') having a lifting surface which, on the side toward the eyelet, ends substantially flush with the proximal surface and, in the direction of the central region, rises by 1 to 5 mm or 1 to 3 mm,
- in the proximal surface, in each case a trough-shaped or groove-like recess (17, 17') in and optionally beyond the region spanned by the bridge-like fastening elements,
- in the side parts, recesses (14, 14') which are continuous from the proximal surface to the distal surface and which extend at least over the region spanned by the bridge-like fastening elements, or which extend at least over the region extending adjacent to the region spanned by the bridge-like fastening elements,
- a separate flat cushion element (24) made of a soft, elastically deformable material and bearing on the distal surface of the shield, wherein the outer circumference of the flat cushion element protrudes beyond the outer circumference of the distal surface of the shield by at least 1 mm on all sides.

13. Shield according to any one of the preceding claims, **characterized in that**, in the side parts, recesses (14, 14') are provided which are continuous from the proximal surface to the distal surface and which extend at least over the region extending adjacent to the region spanned by the bridge-like fastening elements, wherein the shield has a separate flat cushion element (24) made of a soft, elastically deformable material and bearing on the distal surface of the shield, wherein the outer circumference of the flat cushion element protrudes beyond the outer circumference of the distal surface of the shield by at least 1 mm on all sides, wherein the separate cushion element (24) has two eyelets (25, 25') for fastening a retaining strap, wherein the respective eyelet (25, 25') is formed by a bridge-like fastening element (26, 26') which has two pillar portions (28, 28') arranged on the proximal surface (27) of the cushion element (24) and a bridging portion (29) extending between the two pillar portions (28, 28'), wherein the two bridge-like fastening elements (26, 26') of the cushion element (24) are provided such that they protrude through continuous recesses (14, 14') arranged in the side parts of the shield (1) and are thus arranged next to the bridge-like fastening elements (11, 11') of the shield such that the eyelets (25, 25') of the cushion element (24) come to be substantially congruent with the eyelets (10, 10') of the shield (1).

14. Tracheostomy cannula for insertion into a tracheostoma, with a cannula tube and a shield according to any one of Claims 1 to 13.

## Revendications

1. Protection (1) destinée à être montée sur un tube de canule (2) d'une canule de trachéotomie (3) ou sur un connecteur annulaire (4) disposé de façon proximale sur le tube de canule (2), la protection (1) présentant
- une région centrale (5) qui présente un évidement continu (6) servant au guidage du tube de canule (2) et/ou du connecteur (4) à travers celui-ci, et
- deux parties latérales (7, 7') qui se raccordent à deux côtés opposés de la région centrale (5) et s'étendent à partir de là dans la direction radiale jusqu'à l'extrémité respective de la partie latérale (7, 7'),
la protection (1) présentant un côté proximal doté d'une surface proximale (8) qui est tournée à l'opposé du cou du patient lors de l'utilisation, et un côté distal doté d'une surface distale (9) qui est tournée vers le cou du patient lors de l'utilisation, les deux parties latérales présentant respectivement un œillet (10, 10') servant à la fixation d'une bande de retenue,
dans laquelle
l'œillet (10, 10') respectif est formé par un élément de fixation (11, 11') de type pont qui présente deux parties piliers (12, 12') disposées sur la surface proximale (8) de la protection (1), et une partie pont (13) s'étendant entre les deux parties piliers (12, 12'), les deux parties piliers (12, 12') étant disposées à une distance de l'extrémité de la partie latérale (7, 7') respective, **caractérisée en ce que** les deux parties latérales (7, 7') présentent, dans la région enjambée par l'élément de fixation (11, 11'), un évidement continu (14, 14') de la surface proximale (8) à la surface distale (9), lequel fait saillie éventuellement d'environ 1 à 3 mm dans la direction de l'extrémité de la partie latérale (7, 7') au-delà de la région qui est enjambée par l'élément de fixation (11, 11') de type pont.

2. Protection selon la revendication 1, **caractérisée en ce que** la distance entre les côtés intérieurs des parties piliers d'un élément de fixation de type pont se situe dans la plage de 3 à 12 mm et/ou la hauteur libre entre la surface proximale de la protection et le côté intérieur de la partie pont se situe dans la plage de 2 à 5 mm.

3. Protection selon l'une des revendications 1 ou 2, **caractérisée en ce que** la distance des parties piliers à l'extrémité de la partie latérale respective vaut au moins 4 mm, plus préférentiellement au moins 6 mm.

4. Protection selon l'une des revendications précédentes, **caractérisée en ce que** les deux parties piliers de l'élément de fixation respectif et la partie pont s'étendant entre les deux parties piliers présentent une section transversale rectangulaire, une section transversale rectangulaire arrondie, une section transversale ovale ou une section transversale circulaire.

5. Protection selon l'une des revendications précédentes, **caractérisée en ce que** les deux parties piliers de l'élément de fixation respectif forment avec la partie pont s'étendant entre les deux parties piliers respectivement un angle dans la plage de 90 ° à 135 ° ou les deux parties piliers de l'élément de fixation respectif décrivent un arc en plein cintre avec la partie pont s'étendant entre les deux parties piliers.

6. Protection selon l'une des revendications précédentes, **caractérisée en ce que** les deux parties piliers de l'élément de fixation respectif se situent à angle droit sur la surface proximale ou suivant un angle qui diffère de +/- 45 ° de l'angle droit.

7. Protection selon l'une des revendications précédentes, **caractérisée en ce que** la surface distale des parties latérales s'étend sensiblement parallèlement à la surface proximale de la partie latérale respective.

8. Protection selon l'une des revendications précédentes, **caractérisée en ce que** la surface latérale qui s'étend antre la surface distale et la surface proximale d'une partie latérale s'étend, à l'extrémité de la partie latérale, sur la largeur de la protection sur une trajectoire arquée et/ou se prolonge, sans former d'arête, de manière continue dans la surface distale et sélectivement également dans la surface proximale de la partie latérale respective.

9. Protection selon l'une des revendications précédentes, **caractérisée en ce que** la protection est constituée d'une matière souple, déformable élastiquement ou d'une matière dure, à stabilité de forme ou d'une part d'une matière souple, déformable élastiquement et d'autre part d'une matière dure, à stabilité de forme.

10. Protection selon l'une des revendications précédentes, **caractérisée en ce que** les parties latérales de la protection sont formées de manière plate et présentent de préférence une épaisseur de matière dans la plage de 1,5 mm à 4 mm.

11. Protection selon l'une des revendications précédentes, **caractérisée en ce que** la surface distale dans la région centrale présente une portion de courbure concave de façon proximale dans la direction longitudinale de la protection et/ou une portion de courbure concave de façon proximale perpendiculairement à la direction longitudinale de la protection.

12. Protection selon l'une des revendications précédentes, **caractérisée en ce que** la protection présente au moins l'un des dispositifs suivants :
- dans la région centrale, un dispositif de suspension pour le montage à cardan (15) sur le tube de canule ou sur un connecteur d'une canule de trachéotomie,
- sur la surface proximale entre l'œillet et la région centrale, un élément de soulèvement (16, 16') doté d'une surface de soulèvement qui, sur le côté vers l'œillet, se termine sensiblement en affleurement avec la surface proximale et, dans la direction de la région centrale, s'élève de 1 à 5 mm ou de 1 à 3 mm,
- dans la surface proximale, respectivement un évidement (17, 17') en forme de cuvette ou de type rainure dans la région qui est enjambée par les éléments de fixation de type pont et éventuellement au-delà de celle-ci,
- dans les parties latérales, des évidements (14, 14') continus de la surface proximale à la surface distale, lesquels s'étendent au moins sur la région qui est enjambée par les éléments de fixation de type pont, ou lesquels s'étendent au moins sur la région qui s'étend à côté de la région enjambée par les éléments de fixation de type pont,
- en appui contre la surface distale de la protection, un élément de rembourrage (24) plat séparé constitué d'une matière souple, déformable élastiquement, la périphérie extérieure de l'élément de rembourrage plat faisant saillie au-delà de la périphérie extérieure de la surface distale de la protection d'au moins 1 mm de tous côtés.

13. Protection selon l'une des revendications précédentes, **caractérisée en ce que** des évidements (14, 14') continus de la surface proximale à la surface distale sont prévus dans les parties latérales, lesquels s'étendent au moins sur la région qui s'étend à côté de la région enjambée par les éléments de fixation de type pont, la protection présentant un élément de rembourrage (24) plat séparé en appui contre sa surface distale, lequel est constitué d'une matière souple, déformable élastiquement, la périphérie extérieure de l'élément de rembourrage plat faisant saillie au-delà de la périphérie extérieure de la surface distale de la protection d'au moins 1 mm de tous côtés, l'élément de rembourrage séparé (24) présentant deux œillets (25, 25') servant à la fixation d'une bande de retenue, l'œillet respectif (25, 25') étant formé par un élément de fixation (26, 26') de type pont qui présente deux parties piliers (28, 28') disposées sur la surface proximale (27) de l'élément de rembourrage (24) et une partie pont (29) s'étendant entre les deux parties piliers (28, 28'), les deux éléments de fixation (26, 26') de type pont de l'élément de rembourrage (24) étant prévus de telle sorte qu'ils font saillie à travers des évidements continus (14, 14') disposés dans les parties latérales de la protection (1) et sont ainsi disposés à côté des éléments de fixation (11, 11') de type pont de la protection de telle sorte que les œillets (25, 25') de l'élément de rembourrage (24) viennent sensiblement en coïncidence avec les œillets (10, 10') de la protection (1).

14. Canule de trachéotomie destinée à être introduite dans un trachéostome, comportant un tube de canule et une protection selon l'une des revendications 1 à 13.
